# EUROPÄISCHE PATENTANMELDUNG

(11) **EP 1 445 309 A1**
(43) Veröffentlichungstag der Anmeldung: **11.08.2004**
(21) Anmeldenummer: 03029265.0
(22) Anmeldetag: 20.12.2003
(51) Int. Cl.: C12M 1/22

(54) **Verfahren und Vorrichtung zum Ent- und Beladen von mit Petrischalen beladenen Magazinen**

(30) Priorität: 09.01.2003 DE 10301446
(71) Anmelder: A.I.D. Autoimmun Diagnostika GmbH, 72479 Strassberg (DE)
(72) Erfinder: Schöllhorn, Volkmar, 72458 Albstadt-Ebingen (DE); Mayer, Dietmar, 72336 Balingen (DE)
(74) Vertreter: Patentanwälte Ruff, Wilhelm, Beier, Dauster & Partner

(57) **Zusammenfassung**

Bei einem Verfahren zum Ent- und Beladen wenigstens eines mit mindestens einer Petrischale bestückbaren Magazins, wobei die Petrischale ein einen Boden und eine zylindrische Seitenwand aufweisendes schalenförmiges Unterteil und einen lose daraufliegenden Deckel besitzt, wird zunächst eine unterste Petrischale aus einem ersten Magazin seitlich entnommen, wobei das Unterteil der Petrischale mindestens an ihrem Boden erfasst wird. Die Petrischale wird nach Gebrauch von unten durch einen offenen Magazinboden des ersten oder eines zweiten Magazins wieder eingeführt. Eine Ent- und Beladevorrichtung für mit einem Deckel 12d versehene Petrischalen 12 besitzt wenigstens ein Magazin 16, 17, das mit Petrischalen bestückbar ist und einen Schlitten 52 mit wenigstens einem Support 54 zur Aufnahme der Petrischale 12, wobei das Magazin 16, 17 eine Bodenöffnung 25 aufweist mit einem lichten Durchmesser, der größer ist als der Außendurchmesser des Supports 54 und der Petrischale 12. Es ist ferner eine Seitenöffnung 27 vorgesehen, die zumindest in Höhe der untersten Petrischale 12 eine Höhenabmessung aufweist, die in axialer Richtung des Magazins 16, 17 größer ist als die Gesamthöhe aus Petrischale und Support 54.

## Beschreibung

Die Erfindung betrifft ein Verfahren und eine Vorrichtung zum Ent- und Beladen wenigstens eines mit mindestens einer Petrischale bestückbaren Magazins. Die Petrischale besitzt ein schalenförmiges Unterteil mit einem Boden und einem zylindrischen Seitenrand und einen lose auf dem Unterteil aufliegenden Deckel mit einem den Seitenrand nach unten übergreifenden Rand.

Bei vielen biologischen Untersuchungen werden Petrischalen als Kultivierungsgefäß für biologisches Material, insbesondere Zellmaterial eingesetzt. Das biologische Material befindet sich dabei auf einem Nährboden aus Nährmedium, insbesondere Aggar. Nach einer bestimmten Kultivierungsphase des biologischen Materials, beispielsweise einem Anwachsen von Bakterienkulturen, wird es untersucht. Dazu wird die Petrischale in eine Auswerteeinrichtung, beispielsweise in eine Bildauswerteeinrichtung befördert. Da in einem mikrobiologischen Labor viele Petrischalen mit auszuwertendem biologischem Material anfallen, besteht der Bedarf, dass der Transport zur bzw. von der Auswerteeinrichtung und die Auswertung im wesentlichen automatisiert erfolgt.
Es ist bekannt, Petrischalen in Magazinen übereinander zu stapeln, die jeweilige auszuwertende Petrischale herauszunehmen, in eine Auswerteeinheit zu befördern und danach gegebenenfalls in ein anderes Magazin abzulegen. Zum Transport der Petrischalen in die Auswerteeinrichtung sind Greifer bekannt, die die oberste Petrischale greifen, in die Auswerteeinheit legen und zurück in ein Ablage-Magazin befördern. Bei solchen Transporteinrichtungen ist eine exakte Steuerung des Greifers notwendig, da die genaue Position der obersten Petrischale des Stapels angesteuert werden muss, um diese zu greifen.

### Aufgabe und Lösung

Aufgabe der Erfindung ist es, ein Verfahren und eine Vorrichtung der eingangs erwähnten Art zu schaffen, die eine einfachere und gegebenenfalls schnellere Beförderung von Petrischalen mit einer kompakten Vorrichtung ermöglichen.

Diese Aufgabe wird mit einem Verfahren mit den Merkmalen des unabhängigen Anspruchs 1 und einer Ent- und Beladevorrichtung mit den Merkmalen des unabhängigen Anspruchs 11 gelöst. Weiterbildungen der Erfindung sind in den Unteransprüchen dargestellt und werden im folgenden näher erläutert.

Das Verfahren zeichnet sich dadurch aus, dass die unterste Petrischale aus einem ersten Magazin seitlich entnommen wird, wobei das Unterteil der Petrischale mindestens an ihrem Boden erfasst wird. Nach Gebrauch wird die Petrischale von unten durch einen offenen Magazinboden des ersten oder eines anderen Magazins eingeführt. Alternativ kann die Petrischale beim Entnehmen auch zusätzlich an ihrem Seitenrand erfasst werden.

Aus dem Stand der Technik ist es bekannt, die oberste Petrischale mittels eines Greifers aus dem Magazin zu nehmen und nach Gebrauch wieder von oben in ein anderes Magazin zu legen. Die Magazine sind meist mit vielen übereinander zu stapelnden Petrischalen bestückbar und auch bestückt und weisen eine beträchtliche Bauhöhe auf. Deshalb ist ein Greifer notwendig, der diese Bauhöhe überwindet. Eine Vorrichtung, die derart aufgebaut ist, ist relativ sperrig und schwer. Bei einer Ent- und Beladevorrichtung zur Durchführung des erfindungsgemäßen Verfahrens hingegen kann ein hoch auslegender Greifer entfallen, da die unterste Petrischale seitlich entnommen und von unten wieder eingeführt wird. Dementsprechend ist die Ent- und Beladevorrichtung relativ flach und kompakt.

Das erfindungsgemäße Verfahren besitzt gegenüber den aus dem Stand der Technik bekannten Verfahren einen einfacheren Verfahrensablauf und ist damit weniger störanfällig. Während bei den Verfahren, die die Petrischale von oben herausnehmen, die Ladehöhe der Petrischalen im Magazin bestimmt werden muss, damit sich der Greifer an diese Position bewegen kann, kann dieser Schritt beim erfindungsgemäßen Verfahren entfallen, da sich die unterste Petrischale immer an gleicher Position befindet. Ebenso verhält es sich beim Ablegen der Petrischale nach Gebrauch. Bei den aus dem Stand der Technik bekannten Verfahren gibt es prinzipiell zwei Möglichkeiten; entweder bewegt sich der Greifer ans obere Ende des Magazins und lässt die Petrischale ins Magazin fallen, oder es wird wiederum die Stapelhöhe bestimmt und der Greifer bewegt sich an diese Position. Die erste Möglichkeit birgt die Gefahr, dass, falls das Magazin eine geringere Stapelhöhe an Petrischalen aufweist, die abzulegende Petrischale aus einer beträchtlichen Höhe ins Magazin fällt und dabei beschädigt oder gar zerstört wird, mit der Folge, dass möglicherweise biologisches Material in die Umgebung gelangt. Bei der zweiten Möglichkeit ist, wie erwähnt, wiederum eine aufwendige Positionsbestimmung notwendig. Das erfindungsgemäße Verfahren kann in Bereichen eingesetzt werden, die hohe Anforfderungen an die Prozesskontrolle bzw. Validierung stellen, beispielsweise in der klinischen Mikrobiologie oder in der Lebensmittelindustrie.

Als Gebrauch der Petrischale im Sinne der Anmeldung wird insbesondere eine Auswertung des auf dem Nährboden befindlichen biologischen Materials verstanden. Beim biologischen, insbesondere mikrobiologischen Material handelt es sich vorzugsweise um Keime aus Zellmaterial, beispielsweise Bakterienkulturen. Die Auswertung kann beispielsweise eine Bildauswertung sein, bei der ein Schwarz-weiß- oder Farbbild vom biologischen Material gemacht wird. Beispielsweise kann aufgrund der Farbe von Keimen auf deren Identität und aufgrund der flächigen Ausdehnung auf deren Aktivität geschlossen werden.

Bei einer Weiterbildung der Erfindung ist ein Support bzw. eine Aufnahmeelement eines Schlittens vorgesehen, der zum Entnehmen der Petrischale von unten durch den offenen Magazinboden einfährt und dabei vorzugsweise Einlaufschrägen aufweisende, öffenbare Sperren am Magazin öffnet. Die Sperren schnappen spätestens nach der Entnahme der untersten Petrischale wieder zurück. Dadurch wird gewährleistet, dass die sich über der untersten Petrischale befindlichen Petrischalen nicht durch den offenen Magazinboden herausfallen. Die Sperren, insbesondere die an einer Entnahme- bzw. Seitenöffnung des Magazins angeordneten Sperren, bleiben vorzugsweise so lange geöffnet, bis die unterste Petrischale vollständig aus dem Magazin entnommen ist und schnappen dann zurück, bevor die darüber befindliche Petrischale eine Bodenöffnung des Magazinbodens erreicht hat und möglicherweise herausfällt oder durch die zurückschnappenden Sperren eingeklemmt wird. Vorzugsweise sind vier Sperren vorgesehen. Diese können paarweise ausgebildet und angeordnet sein, insbesondere spiegelbildlich zueinander.

Das Einführen der Petrischale von unten erfolgt vorzugsweise derart, dass die Petrischale, insbesondere ihr Deckel beim Einführen von unten die Sperren, vorzugsweise alle Sperren, öffnet und diese nach dem Einführen wieder zurückschnappen. Falls sich im Magazin bereits wenigstens eine Petrischale befindet kann diese durch die einzuführende Petrischale nach oben angehoben werden und die Sperren bleiben so lange zumindest teilweise geöffnet, bis der Support vorzugsweise nach unten aus dem Magazin herausgefahren wird. Die Sperren können in einer im wesentlichen radialen Richtung, vorzugsweise auf einer kreisbogenförmigen Bahn des Magazins nach außen gedrückt werden und nach innen zurückschnappen. Es ist auch möglich, die Sperren in axialer Richtung des Magazins in Einführrichtung nach oben zurückzudrücken, so dass sie nach unten zurückschnappen.

Die Sperren können schwenkbeweglich zwischen einer Öffnungs- und Schließposition am Magazin mittels einer Schwenklagerung gelagert sein. In Schließposition können die Sperren ein sich axial zu einer Magazinlängsachse verlaufende, sich verjüngende Durchlassöffnung für die Petrischalen bilden. Die lichte Durchgangsöffnung des Magazinbodens bei geschlossenen Sperren ist bevorzugt kleiner oder anders geformt als die Flächenausdehnung des Petrischalen-Deckels, so dass alle Sperren beim Einfahren zurückgedrückt werden. Es kann weiterhin die lichte Durchgangsöffnung in Schließposition der Sperren an ihrem in der vertikalen Einführrichtung des Supports vorderen (unteren) Ende größer sein, als der Außendurchmesser des Supports und an ihrem hinteren (oberen) Ende kleiner sein als der Außendurchmesser des Supports. Dadurch werden die Sperren erst nach einem bestimmten Einfahrweg des Supports zurückgedrückt.

Besonders bevorzugt sind die Sperren als Klinken ausgebildet. Es ist auch möglich elastische Elemente, beispielsweise in Form von Federn als Sperren vorzusehen. Es ist möglich, zwei Arten von Klinken mit unterschiedlichen Funktionen vorzusehen. Bevorzugt treten die an einer Seitenöffnung des Magazins befindlichen Klinken sowohl beim seitlichen Entnehmen der Petrischale als auch beim Einführen der Petrischale von unten in Funktion, d.h. werden zurückgedrückt und schnappen zurück. Die hinteren Klinken des Magazins, die vorzugsweise den vorderen Doppelfunktions-Klinken gegenüberliegend angeordnet sind, treten lediglich beim Einführen der Petrischale von unten in Funktion, werden also bei der seitlichen Entnahme der Petrischale nicht zurückgedrückt.

Jede Klinke kann mindestens eine Einlaufschräge aufweisen, wobei vorzugsweise die im Bereich der Seitenöffnung bzw. Entnahmeöffnung angeordneten Klinken eine Einlaufschräge für das Einführen von unten und eine Auslaufschräge für die seitliche Entnahme aufweisen. Die Klinken können an ihrem in Einführrichtung gesehen hinteren (oberen) Ende im wesentlichen senkrecht zur Magazinlängsachse verlaufende, horizontale Oberseiten bzw. Abschlüsse aufweisen, die in Schließposition der Klinken eine Auflagefläche für die unterste Petrischale des Magazins bilden.

Die Sperren können Rückstellelemente, insbesondere Federelemente aufweisen, um ein selbsttätiges Zurückschnappen in ihre Schließposition zu ermöglichen. Jeder Sperre kann ein insbesondere am Magazin ausgebildeter Anschlag, insbesondere Steg, zur Begrenzung der Schwenkbewegung in einer Sperren-Schließrichtung zugeordnet sein. Der Anschlag erstreckt sich vorzugsweise im wesentlichen senkrecht zur Sperren-Schließrichtung und wirkt mit an den Sperren ausgebildeten Stoßflächen in deren Schließposition zusammen.

Der Support zur Aufnahme der Petrischale kann zumindest einen Bereich aufweisen, dessen Außenabmessungen kleiner ist als der lichte Öffnungsquerschnitt der Bodenöffnung des Magazins und größer ist, als die von den Sperren in Schließposition gebildete Durchlassöffnung. Der Support passt also durch die Bodenöffnung des Magazins, jedoch nicht durch die Durchgangsöffnung der Sperren, so dass mindestens eine, vorzugsweise zwei Sperren beim Einfahren des Supports zurückgedrückt werden. Beim Support kann sich an einen Abschnitt bzw. Bereich großer Außenabmessung, insbesondere Breite, ein Abschnitt mit gegenüber der großen Außenabmessung kleineren Querabmessung anschließen, wobei diese kleine Querabmessung kleiner ist, als die von den Sperren gebildete Durchlassöffnung. Dadurch ist es möglich, dass der Support bei Einführen von unten durch den Abschnitt mit großer Außenabmessung insbesondere die an der Entnahme- bzw. Seitenöffnung angeordneten Sperren zurückdrückt, während der Abschnitt mit der kleineren Querabmessung zwischen den insbesondere gegenüberliegend angeordneten Sperren hindurchfährt, ohne diese zurückzudrücken.

Der Support weist vorzugsweise eine mittige Durchbrechung bzw. Öffnung auf. Insbesondere ist er als relativ schmales Materialband ausgebildet, das eine äußere und eine die Durchbrechung definierende innere Kontur bildet. Es ist jedoch auch möglich, den Support ohne mittige Durchbrechung beispielsweise in Form eines Tellers auszubilden. Der Support kann Zentrierelemente zur Sicherung der Petrischale gegen seitliches Abrutschen vom Support aufweisen. Die Zentrierelemente erstrecken sich vorzugsweise im wesentlichen senkrecht zu einer Schlittenlängsachse und sind insbesondere derart angeordnet, dass sie eine Umgreifung für eine Petrischale bilden. Der Durchmesser der Umgreifung ist also vorzugsweise größer als der Außendurchmesser des Petrischalen-Unterteils. Die Zentrierelemente sind insbesondere einstückig mit dem Support verbunden. Vorzugsweise ist an jedem Zentrierelement eine Einlaufschräge vorgesehen. Die Zentrierelemente können beispielsweise die Form von Nasen oder Stiften haben.

Nach dem Entnehmen kann die Petrischale auf einen durch eine Öffnung des Supports passenden, den Boden der Petrischale erfassenden Tisch abgesetzt werden. Die größte Außenabmessung des Tisches ist vorzugsweise kleiner als die größte Außenabmessung der Durchbrechung des Supports und kleiner als der lichte Abstand von Randbegrenzungen einer Schiebeeinrichtung.

Die Petrischale kann von einer den Deckel erfassenden Schiebeeinrichtung seitlich verfahren, insbesondere verschoben werden. Die Schiebeeinrichtung kann Randbegrenzungen aufweisen, deren lichter Abstand größer ist als der Außendurchmesser des Unterteils der Petrischale und kleiner ist als der Außendurchmesser des Deckels. Das Petrischalen-Unterteil kann also durch den Öffnungsquerschnitt der Randbegrenzungen hindurchfallen, während der Deckel fixiert und zentriert wird. Im Bereich der Anschlagelemente kann ein Vorsprung vorgesehen sein, der in Schieberichtung in den Abstandsbereich der Anschlagelemente hineinragt und eine Erfassungsstelle zwischen Schiebeeinrichtung und Deckel bildet. Die Schiebeeinrichtung kann als den Deckel der Petrischale umgreifender Ring ausgebildet sein.

Bei einer Weiterbildung der Erfindung weist das Magazin wenigstens ein Sicherungselement zur Sicherung der Petrischale gegen seitliches Herausfallen aus der Seitenöffnung auf. Das Sicherungselement kann als elastisches Element ausgebildet sein, das im Bereich der Seitenöffnung angeordnet ist und in Höhe der Petrischale in den Öffnungsquerschnitt der Seitenöffnung hineinragt. Das elastische Element kann beispielsweise eine Feder sein.

Der Innendurchmesser des Deckelrandes der Petrischale ist vorzugsweise größer als der Außendurchmesser des Unterteils der Petrischale. Das Verhältnis des Deckelrandinnendurchmessers zum Unterteilaußendurchmesser kann im Bereich von 1,01 bis 1,06, insbesondere 1,03 bis 1,05 liegen. Bevorzugt ist ein den Seitenrand des Petrischalen-Unterteils außen überlappender Deckelrand vorgesehen. Der Boden des Petrischalen-Unterteils ist vorzugsweise eben.

Die Petrischale kann nach dem Entnehmen, vorzugsweise nach einem Abheben des Deckels, in eine Position zum Gebrauch, insbesondere zur Bildauswertung gefahren werden. Zum Abheben des Deckels kann eine Deckelabnehmeinrichtung vorgesehen sein. Die Bildauswerteeinrichtung kann ein Imageanalyzer sein, vorzugsweise mit einer lichtdurchlässigen Photolichtplatte, einer Kamera und einer Auf- und oder Durchlichteinheit.

Das erste Magazin ist vorzugsweise mit mehreren Petrischalen beladen und eine ausgewertete Petrischale wird von unten in das zweite Magazin eingeführt. Zum Einführen der Petrischale kann der Schlitten unter das zweite Magazin, insbesondere unter ein Belademagazin, fahren. Alternativ ist es möglich, dass das zweite Magazin über den Schlitten fährt.

Bei einer Weiterbildung der Erfindung kann das wenigstens ein Magazin lösbar an der Ent- und Beladevorrichtung angeordnet sein. Es kann eine Positioniereinrichtung zur Positionierung des Magazins vorgesehen sein. Dadurch ist es möglich, dass beim Bestücken der Ent- und Beladevorrichtung mit neuen Magazinen diese immer an die gleiche Position kommen.

Ferner umfasst die Erfindung eine Vorrichtung mit den Merkmalen des unabhängigen Anspruchs 11.

Die erfindungsgemäße Ent- und Beladevorrichtung besitzt wenigstens ein Magazin, das mit Petrischalen bestückbar ist und einen Schlitten mit wenigstens einem Support zur Aufnahme der Petrischale, wobei das Magazin eine Bodenöffnung aufweist mit einem lichten Durchmesser, der größer ist als der Außendurchmesser des Supports und der Petrischale und zumindest in Höhe der untersten Petrischale eine Seitenöffnung aufweist, deren Höhenabmessung in axialer Richtung des Magazins größer ist als die Gesamthöhe aus Petrischale und Teller.

Bezüglich weiterer und näherer Details der Ent- und Beladevorrichtung wird auf die vorgehende Beschreibung und die nachfolgende Beschreibung von Ausführungsbeispielen in Verbindung mit der Zeichnung und den Unteransprüchen verwiesen.

Die vorstehenden und weitere Merkmale der Erfindung gehen außer aus den Ansprüchen auch aus der Beschreibung und den Zeichnungen hervor, wobei die einzelnen Merkmale jeweils sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können. Die Unterteilung der Anmeldung in einzelne Abschnitte sowie Zwischenüberschriften beschränkt die unter diesen gemachten Aussagen nicht in ihrer Allgemeingültigkeit.

### Beschreibung der Zeichnungen

Bevorzugte Ausführungsbeispiele der Erfindung sind in den Zeichnungen dargestellt und werden im folgenden näher erläutert. In den Zeichnungen zeigen:
- Fig. 1: eine Innenansicht eines Automaten zum Petrischalen-Handling von oben her gesehen;
- Fig. 2: eine Vorderansicht eines Magazins einer erfindungsgemäßen Ent- und Beladevorrichtung für Petrischalen;

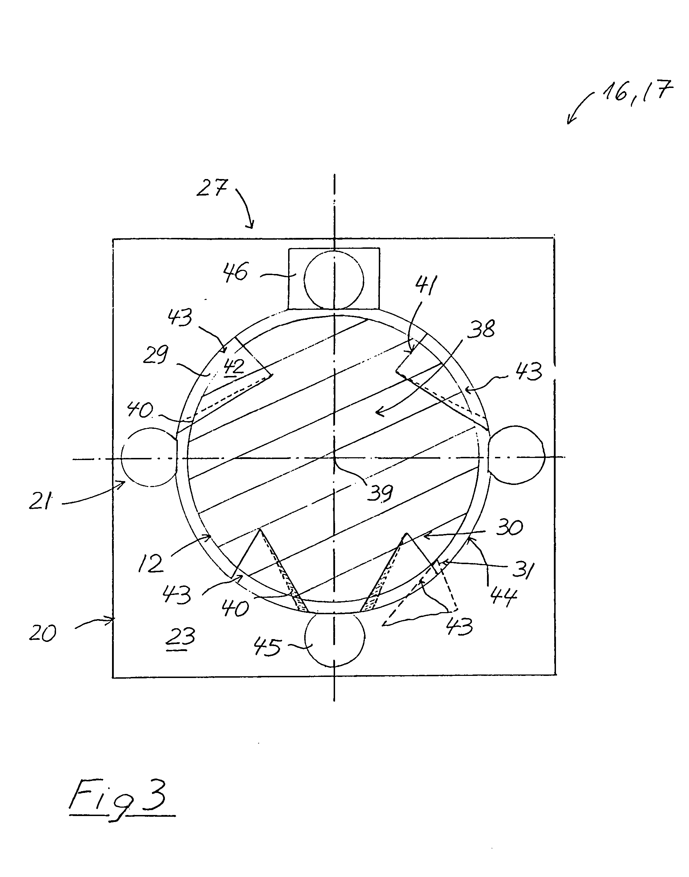

- Fig. 3: eine Draufsicht auf das Magazin von Fig. 2;
- Fig. 4: eine Unteransicht auf das Magazin von Fig. 2;
- Fig. 5: eine Draufsicht auf einen Tandemschlitten der Ent- und Beladevorrichtung;
- Fig. 6: eine Seitenansicht auf eine erfindungsgemäße Deckelabnehmeinrichtung für Petrischalen und
- Fig. 7: eine Draufsicht auf die Deckelabnehmeinrichtung nach Fig. 6.

### Detaillierte Beschreibung der Ausführungsbeispiele

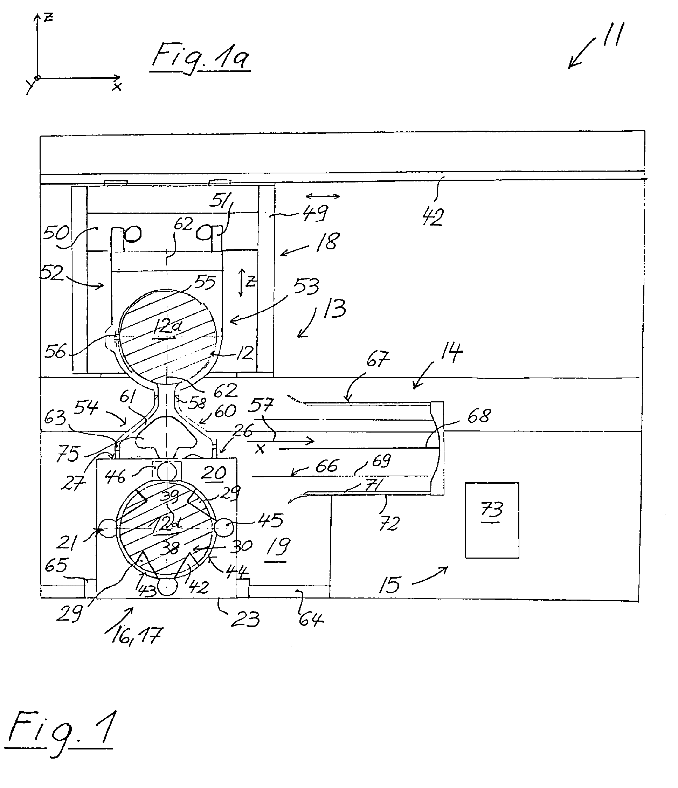

In der Fig. 1 ist ein Ausführungsbeispiel eines Automaten 11 bzw. Roboters für ein automatisches Handling von Petrischalen 12 dargestellt. Der Automat 11 umfasst eine Ent- und Beladevorrichtung 13, eine Deckelabnehmeinrichtung 14 und eine Bildauswerteeinrichtung 15 für Petrischalen 12. Es kann ein Automatengehäuse vorgesehen sein, das die Ent- und Beladevorrichtung 13, die Deckelabnehmeinrichtung 14 und die Bildauswerteeinrichtung 15 umschließt.

Die Ent- und Beladevorrichtung 13 dient zur Durchführung des erfindungsgemäßen Verfahrens zum Ent- und Beladen wenigstens eines mit einer Petrischale 12 bestückbaren Magazins. Die Ent- und Beladevorrichtung 13 umfasst ein mit wenigstens einer Petrischale 12 bestücktes Entlademagazin 16, ein Belademagazin 17, gegebenenfalls weitere Magazine, eine Transporteinrichtung 18 und eine Positioniereinrichtung 19.

Im beschriebenen Ausführungsbeispiel besitzt die Petrischale 12 ein Unterteil 12a mit Boden 12b und Seitenrand 12c sowie einen Deckel 12d mit das Unterteil übergreifendem Deckelrand 12e. Die Petrischale 12 ist vorzugsweise aus fotolichtdurchlässigem Plexiglas hergestellt. Das Unterteil 12a der Petrischale 12 dient zur Aufnahme eines Nährmediums für biologisches Material und des biologischen Materials selber. Als Nährmedium kann beispielsweise Aggar verwendet werden. Das Unterteil 12a besitzt einen kreisrunden Querschnitt. Der Deckel 12d besitzt ebenfalls einen kreisrunden Querschnitt und liegt lose auf dem Unterteil 12a auf. Im beschriebenen Ausführungsbeispiel ist der Innendurchmesser des Deckelrandes 12e größer als der Außendurchmesser des Unterteils 12a. Das Verhältnis des Deckelinnendurchmessers zum Unterteilaußendurchmessers liegt vorzugsweise im Bereich von 1,03 bis 1,05. Der Seitenrand 12c des Unterteils wird also von dem nach unten ragenden Deckelrand 12e des Deckels 12d überlappt. Der Deckelrand reicht ungefähr bis zur halben Höhe des Seitenrandes 12c herunter. Der Boden 12b der Petrischale ist eben ausgebildet. Das Verhältnis der Gesamthöhe der Petrischale 12; d.h. das Unterteil 12a mit aufgelegtem Deckel 12d; zum Deckelaußendurchmesser liegt vorzugsweise im Bereich von 0,1 bis 0,3. Das Gesamtgewicht der Petrischale 12 samt Nährmittel und biologischem Material ist so groß, dass ein nachfolgend noch näher beschriebener Ent- und Beladevorgang eines mit Petrischalen bestückbaren Magazins durchführbar ist.

Es können auch mehrere Entlade- bzw. Belademagazine 16, 17 vorgesehen sein.

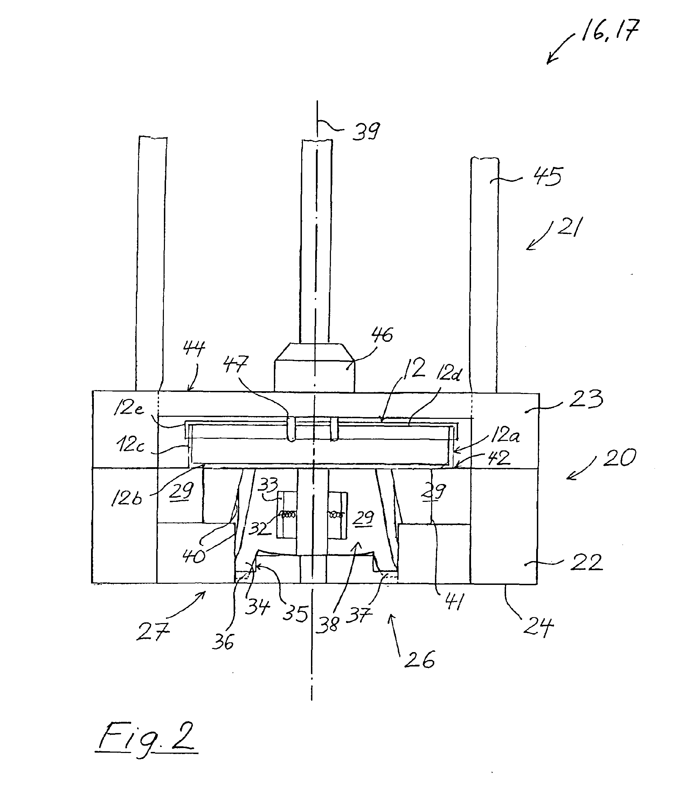

In Fig. 2 ist ein Entlade- bzw. Belademagazin 16, 17 dargestellt. Das Belademagazin 17 ist vorzugsweise identisch mit dem Entlademagazin 16, so dass die Ausführungen die sich auf das Entlademagazin 16 beziehen auch für das Belademagazin 17 gelten. Das Belademagazin 17 befindet sich vorzugsweise neben dem Entlademagazin 16 im Automat 11.

Das Entlademagazin 16 besitzt eine Basis 20 und eine seitliche Stapelführung 21.

Die Basis 20 besteht aus einem Unterteil 22 und einem Oberteil 23. Die Basis 20 ist insbesondere als massiver Metallblock, insbesondere aus Stahl oder Aluminium, hergestellt.

Das Unterteil 22 hat einen Magazinboden 24 mit Bodenöffnung 25, einer Vorderseite 26 mit Seitenöffnung 27, die unter anderem zur Entnahme von Petrischalen dient, und eine am Magazinboden 24 ausgebildete Nut 28 auf der von der Seitenöffnung abgewandten Seite des Magazins.

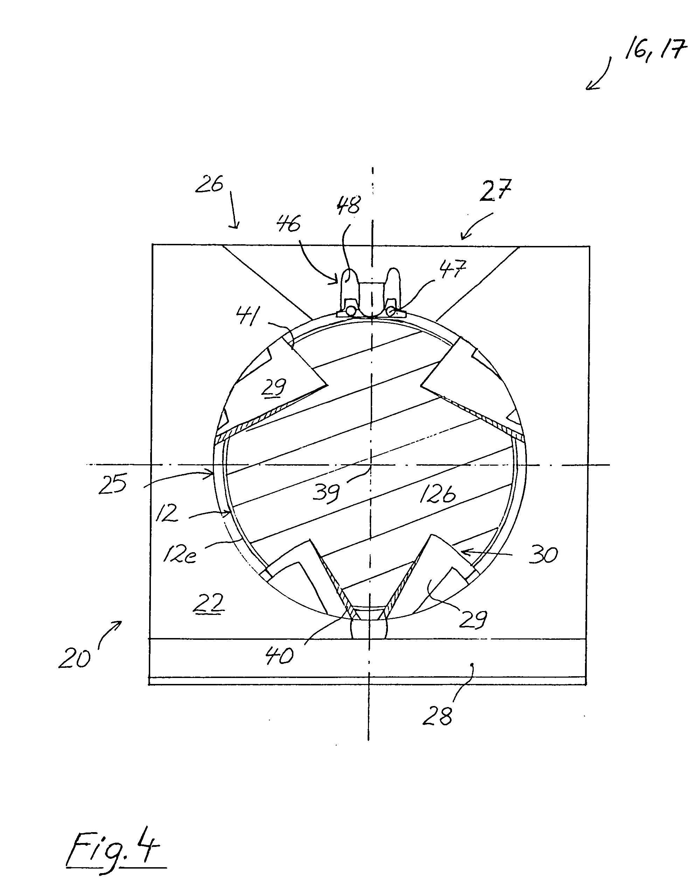

Die Fig. 4 zeigt die Unteransicht des Entlademagazins mit der Bodenöffnung 25. Die Bodenöffnung 25 hat einen kreisrunden Querschnitt und ist zur Vorderseite 26 hin trichterförmig geöffnet. Der Durchmesser der Bodenöffnung 25 ist größer als der Außendurchmesser des Deckels 12d der Petrischale 12. Es sind auch andere Querschnitte der Bodenöffnung 25 möglich, beispielsweise ein rechteckiger, quadratischer o.dgl. jedenfalls muss der Öffnungsquerschnitt der Bodenöffnung 25 größer sein als der Außendurchmesser des Petrischalen-Deckels 12d oder eines nachfolgend näher beschriebenen Supports 54, damit die Petrischale 12 bzw. der Support 54 von unten her ins Magazin gelangen kann. Die Seitenöffnung 27 hat einen rechteckigen Querschnitt. Auch hier sind andere Querschnitte möglich. Die Breite der Seitenöffnung 27 ist größer als der Außendurchmesser des Petrischalen-Deckels 12d. Die Höhe der Seitenöffnung 27 ist größer als die Gesamthöhe der Petrischale 12, damit die Petrischale 12 seitlich entnommen werden kann. Die Nut 28 verläuft parallel zur Seitenöffnung 27 vorzugsweise über die gesamte Breite des Magazinbodens 24.

Am Basis-Unterteil 22 des Entlademagazins 16 sind, der Bodenöffnung 25 zugeordnet, Sperren in Form von Klinken 29 befestigt. Die Klinken 29 sind schwenkbar zwischen einer Schließposition 30 und einer Öffnungsposition 31 gelagert. In der Schließposition 30 ragen die Klinken 29 teilweise in den Öffnungsquerschnitt der Bodenöffnung 25 hinein und bilden ihrerseits eine von der Kreisform abweichende Durchlassöffnung 38. In der Öffnungsposition 31 befinden sich die Klinken 29 nach außen zurückgedrückt zumindest teilweise in einem imBasis-Unterteil 22 gebildeten Hohlraum und geben die Bodenöffnung 25 frei. Es sind Rückstellelemente 32 in Form von Federn, insbesondere Zugfedern vorgesehen, die ein selbsttätiges Zurückschnappen der Klinken 29 von ihrer Öffnungsposition 31 in die Schließposition 30 ermöglichen. Vorzugsweise ist jeder Klinke 29 eine solche Zugfeder zugeordnet. Die Zugfedern sind einerseits mit dem Basis-Unterteil 22 und andererseits mit einem an der Klinke 29 ausgebildeten Bolzen 33 verbunden. An der Unterseite der Klinken 29 sind Führungsnasen 34 vorgesehen, die bei der Klinken-Verschwenkung zwischen der Schließposition 30 und der Öffnungsposition 31 in eine kreisbogenförmig ausgebildete Führungsbahn 35 am Basis-Unterteil 22 eingreifen. An der Vorderseite, der Bodenöffnung 25 zugewandten Seite, der Führungsbahn 35 ist ein Anschlag 36 in Form eines Steges ausgebildet, der mit einem an der Führungsnase 34 ausgebildeten Klinkenanschlag 37 zusammenwirkt und damit die Schließposition 30 der Klinke 29 vorgibt. Die Durchlassöffnung 38 der Klinken 29 verjüngt sich in axialer Richtung zu einer Magazinlängsachse 39 weg von der Bodenöffnung 25. Der lichte Durchmesser der Durchlassöffnung 38 ist kleiner als der Außendurchmesser des Deckels 12d der Petrischale 12.

Es sind vorzugsweise vier Klinken 29 vorgesehen, von denen sich zwei im Bereich der Seitenöffnung 27 und zwei insbesondere gegenüberliegend im hinteren Bereich des Basisunterteils 22 befinden. Die Klinken 29 besitzen axial zur Magazinlängsachse 39 ausgerichtete Einlaufschrägen 40. Die im Bereich der Seitenöffnung 27 angeordneten Klinken 29 besitzen zusätzlich eine Auslaufschräge 41 für das seitliche Entnehmen der Petrischale 12. Die Klinken 29 besitzen eine im wesentlichen senkrecht zur Magazinlängsachse 39 ausgerichtete ebene Oberseite 42.

Die Oberseiten 42 haben in Schließposition 30 der Klinken 29 eine Dreiecksform oder die Form unregelmäßiger Vierecke. Die Oberseiten 42 bilden zusammen eine Auflagefläche 43, auf der die unterste Petrischale 12 des Entlademagazins 16 aufliegt.

Das Basisoberteil 23 ist einstückig mit dem Basisunterteil 22 verbunden. An seiner Vorderseite befindet sich der obere Teil der Seitenöffnung 27. Das Basisoberteil 23 besitzt ferner eine Oberteilöffnung 44, die in entgegengesetzt zur Bodenöffnung 25 liegt. Die Oberteilöffnung 44 hat einen kreisrunden Querschnitt. Vorzugsweise entspricht der lichte Durchmesser der Oberteilöffnung 44 dem lichten Durchmesser der Bodenöffnung 25.

Die seitliche Stapelführung 21 dient zur seitlichen Fixierung des Petrischalenstapels im Entlademagazin 16. Als Stapelführung 21 sind im beschriebenen Ausführungsbeispiel insbesondere vier vorzugsweise in regelmäßigen Abständen um die Oberteilöffnung 44 angeordnete Stäbe 45 mit vorzugsweise kreisrundem Querschnitt vorgesehen. Durch die Höhe der Stäbe 45 wird die Gesamtstapelhöhe der Petrischalen 12 im Entlademagazin 16 vorgegeben. Die Stäbe 45 ragen teilweise in den Öffnungsquerschnitt der Oberteilöffnung 44 hinein. Der hineinragende Teil der Stäbe ist dabei als Einlaufschräge von unten ausgebildet. Der an der Vorderseite der Basis 10 oberhalb der Seitenöffnung 27 angeordnete Stab 45 hat an seiner Unterseite einen Basisabschnitt 46. Der Basisabschnitt 46 hat insbesondere die Form eines Quaders und dient zur Aufnahme von Sicherungselementen 47, die die unterste Petrischale 12 gegen seitliches Herausfallen aus der Seitenöffnung 27 sichern. Die Sicherungselemente 47 ragen dafür teilweise in den Öffnungsquerschnitt der Seitenöffnung 27 hinein. Die Sicherungselemente 47 sind vorzugsweise als an einem Ende festgelegte nach unten ragende Wendelfedern ausgebildet, die beim Entnehmen der Petrischale 12 zurückgedrückt werden und sich dabei in eine an der Unterseite des Basisabschnitts 46 ausgebildete, ungefähr an die Form der Federn angepasste Ausnehmung 48 legen.

In der Fig. 1 ist schematisch die Transporteinrichtung 18 dargestellt. Die Transporteinrichtung ist Teil der Ent- und Beladevorrichtung und dient insbesondere zum Transport der Petrischale 12 vom Entlademagazin 16 zur Deckelabnehmerrichtung 14 und von dort zur Bildauswerteeinrichtung 15 und zurück in das Belademagazin 17. Alternativ sind auch andere Transportwege möglich, beispielsweise vom Entlademagazin 16 zu einer Gebrauchseinrichtung, die nicht die Bildauswerteeinrichtung sein muss und zurück zum Belademagazin 17. Die Transporteinrichtung 18 umfasst ein Fahrgestell 49 für die Bewegung der Petrischale in eine horizontale x-Richtung, ein Fahrgestell 50 für die Bewegung in eine horizontale z-Richtung und ein Fahrgestell 51 für die Bewegung in eine zur Blattebene von Figur 1 senkrechte vertikale y-Richtung (vgl. Figur 1a).

Das Fahrgestell 49 für die Bewegung in x-Richtung ist mittels Rollen auf zwei Laufschienen 42 des Automaten 11 verfahrbar. Es ist mittels eines Antriebsriemens mit einem als Antrieb ausgebildeten Stellmotor verbunden. Das Fahrgestell 49 für die Bewegung in x-Richtung besitzt ebenfalls zwei Schienen auf denen das Fahrgestell 50 für die Bewegung in z-Richtung mittels Rollen verfahrbar ist. Auch dieses Fahrgestell ist über einen Antriebsriemen mit einem Stellmotor gekoppelt. Das Fahrgestell 50 für die Bewegung in z-Richtung besitzt zwei in vertikaler Richtung angeordnete Schienen, auf denen das Fahrgestell 51 für die Bewegung in y-Richtung mittels Rollen verfahrbar angeordnet ist. Auch dort ist ein Antriebsriemen und ein Stellmotor vorgesehen. Es ist also möglich, jede beliebig Position innerhalb des Automaten 11 anzufahren.

Das Fahrgestell 51 für die Bewegung in y-Richtung besitzt einen Tandemschlitten 52 mit einer Schiebeeinrichtung 53 und einem Support 54.

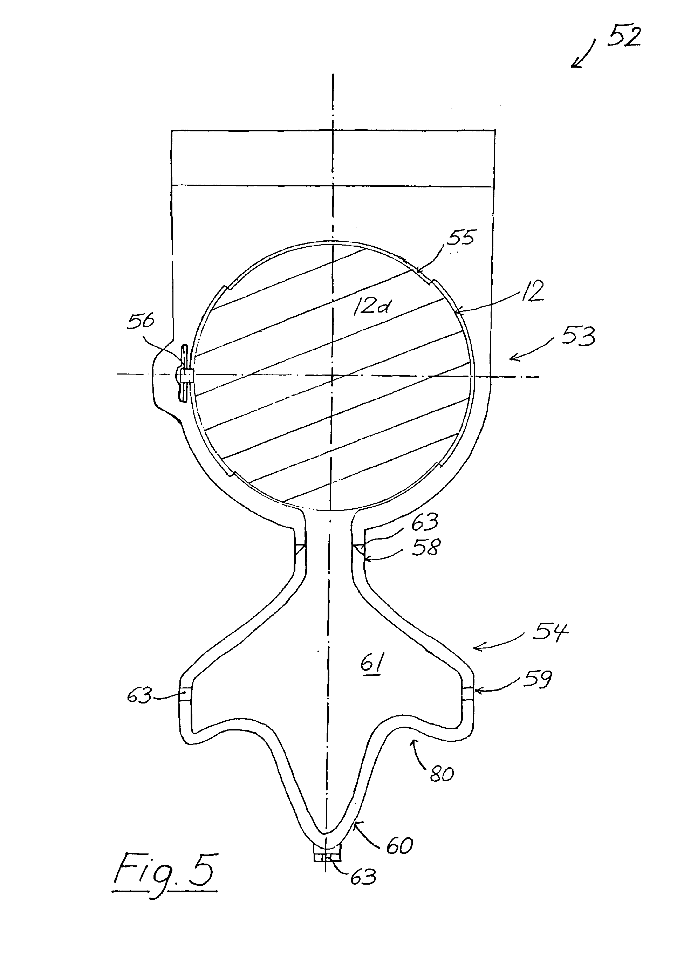

In der Fig. 5 ist der Tandemschlitten 52 dargestellt. Die Schiebeeinrichtung 53 ist im beschriebenen Ausführungsbeispiel als Ring ausgebildet. Der lichte Innendurchmesser des Rings ist größer als der Außendurchmesser des Unterteils 12a der Petrischale 12 und insbesondere an Stegbereichen 55 kleiner als der Außendurchmesser des Deckels 12d der Petrischale 12. An der Schiebeeinrichtung 53 ist ein Vorsprung 56 in Form eines Rädchens vorgesehen, das in Schieberichtung in die Querschnittsöffnung der Schiebeeinrichtung 53 hineinragt. Das Rädchen dient zum Erfassen des Deckelrandes 12e des Petrischalen-Deckels 12d. An der nicht dargestellten Unterseite der Schiebeeinrichtung 53 befindet sich ein Einschnitt, der beim Verschieben der Petrischale mit einer nachfolgend näher beschriebenen Führung für das Petrischalen-Unterteil 12a zusammenwirkt.

Der Support 54 ist einstückig und in etwa gleicher Höhe mit der Schiebeeinrichtung 53 verbunden und dient zur Aufnahme und zum Transport der Petrischale 12. Er ist als schmales Materialband, insbesondere aus Stahl, ausgebildet, mit einer charakteristischen äußeren und inneren Kontur. Der Support 54 besitzt einen Übergangsabschnitt 58, der die Verbindung mit der Schiebeeinrichtung darstellt, einen breiten Mittelabschnitt 59, einen annähernd spitz zulaufenden Endabschnitt 60 und eine mittige Durchbrechung 61.

Der Übergangsabschnitt 58 schließt sich insbesondere unmittelbar an die Schiebeeinrichtung 53 an und ist spiegelsymmetrisch und parallel zu einer Schlittenlängsachse 62 ausgerichtet. Am Übergangsabschnitt 58 sind Zentrierelemente 63 in Form von Nasen ausgebildet, die sich in vertikaler y-Richtung, also senkrecht zur Schlittenlängsachse 62 erstrecken. Die Nasen sind in Draufsicht dreieckig und haben eine zur Schlittenlängsachse 62 geneigte Schräge. An den Übergangsabschnitt 58 schließt sich der Mittelabschnitt 59 an. Er besitzt gegenüber dem Übergangsabschnitt 58 eine größere horizontale Querabmessung senkrecht zur Schlittenlängsachse 62. Der Mittelabschnitt 59 ist ebenfalls spiegelsymmetrisch zur Schlitten längsachse 62 ausgebildet und hat die Form von zwei aufgespreizten Flügeln. An der dem Übergangsabschnitt 58 gegenüberliegenden Seite des Mittelabschnitts 59 befinden sich, quasi an der von der Schiebeeinrichtung 53 abweisenden Seite der Flügel, Einbuchtungen 80. Der Mittelabschnitt 59 besitzt ebenfalls in y-Richtung zeigende Nasen. Der Mittelabschnitt 59 geht in den Endabschnitt 60 über. Der Endabschnitt 60 ist in etwa parabelförmig ausgebildet. Er besitzt ebenfalls eine in y-Richtung ausgerichtete Nase. Die Nasen am Übergangsabschnitt 58 am Mittelabschnitt 59 und am Endabschnitt 60 bilden eine Umgreifung für eine aufzunehmende Petrischale 12. Derlichte Durchmesser der Umgreifung ist größer als der Außendurchmesser des Petrischalen-Unterteils 12a. Der Durchmesser der Umgreifung vergrößert sich vertikaler y-Richtung, so dass beim Übernehmen der Petrischale 12 Fehlstellungen mit dem Support 54 ausgeglichen werden können.

Die Positioniereinrichtung 19 dient zum Positionieren des Entlademagazins 16 und des Belademagazins 17 im Automat 11. Die Positioniereinrichtung 19 besitzt eine horizontale Stange 64, die in die Nut 28 am Magazinboden 24 eingreifen kann. Es sind ferner Positionieranschläge 65 vorgesehen, die die genaue Position der Magazine innerhalb des Automaten 11 festlegen.

In der Fig. 1 ist ein Tisch 75 dargestellt, der beim Umsetzen der Petrischale vom Support 54 in die Schiebeeinrichtung 53 verwendet wird. Der Tisch hat eine äußere Kontur, die an die innere Kontur der mittigen Durchbrechung 61 am Support 54 angepasst ist. Die größte Querabmessung des Tisches ist dabei kleiner als die größte Querabmessung des Supports 54, so dass der Support über den Tisch abgesenkt werden kann.

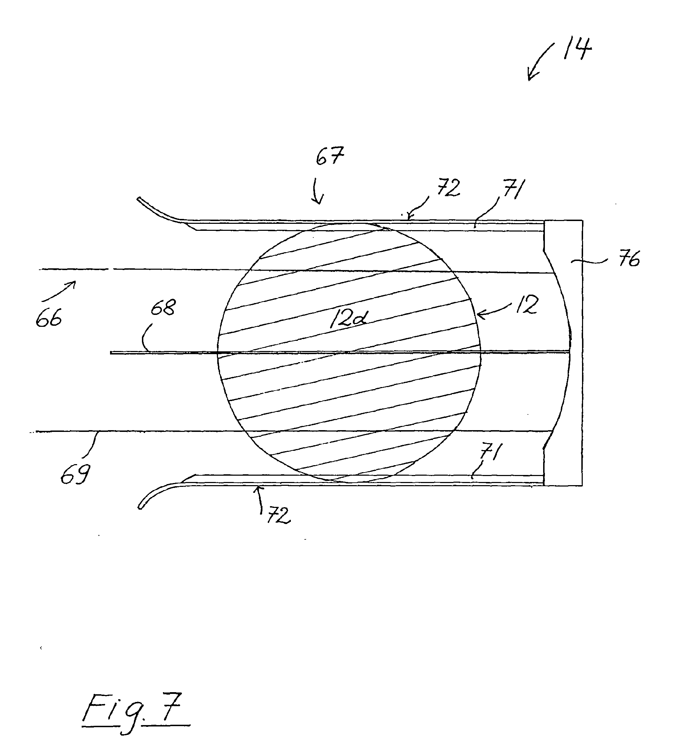

In den Fig. 1, 6 und 7 ist eine Deckelabnehmeinrichtung 14 dargestellt, die zur Durchführung des Verfahrens zum Abnehmen des Deckels einer Petrischale verwendet wird. Die Deckelabnehmeinrichtung 14 besteht im wesentlichen aus einer Unterteilführung 66, einer schräg dazu angeordneten Deckelführung 67 und einem Niederhalter 68.

Die Unterteilführung 66 ist im beschriebenen Ausführungsbeispiel als U-Profil mit zwei in vertikaler y-Richtung ausgerichteten Schenkeln 69 ausgebildet. Der lichte Abstand der beiden Schenkel 69 ist kleiner als der lichte Abstand des Einschnitts an der Unterseite der Schiebeeinrichtung 53. Somit lässt sich die Schiebeeinrichtung 53 und mit dieser die Petrischale auf die Unterteilführung 66 aufschieben. An der in Schieberichtung 57 liegenden Vorderseite der beiden Schenkel 69 ist jeweils eine Unterteilführungs-Einlaufschräge 70 ausgebildet. Der nicht dargestellte Endbereich der Unterteilführung 66 kann gegenüber der Horizontalen in Schieberichtung 57 schräg nach unten ausgerichtet sein.

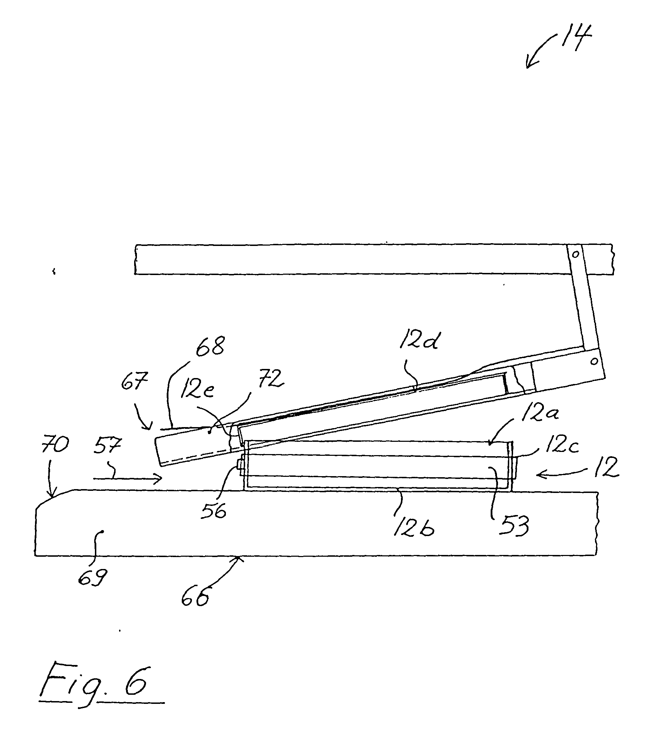

Die Deckelführung 67 besitzt eine Deckeluntergreifungseinrichtung 71 und eine Seitenführung 72. Die Deckelführung 67 besitzt eine gegenüber der Unterteilführung in Pfeilrichtung 57 (Fig. 6) nach oben ausgerichtete Schräge. Die Deckeluntergreifungseinrichtung 71 wird von zwei parallel zur Schieberichtung 57 angeordneten Schienen gebildet. Der lichte Abstand der beiden Schienen ist kleiner als der Außendurchmesser des Petrischalen-Deckels 12d und größer als der Außendurchmesser des Unterteils 12a. Die in Schieberichtung 57 liegenden Vorderkanten der Schienen sind in einer bestimmten Höhe über der Unterteilführung 66 angeordnet. Der lichte Abstand zwischen der Unterteilführung 66 und den Vorderkanten der Schienen ist kleiner als die Höhe des Seitenrandes 12c des Unterteils 12a der Petrischale 12. Somit kann das Petrischalen-Unterteil 12a bei einer der Schieberichtung 57 entgegengesetzten Bewegung mit seinem Seitenrand 12c an den Deckelrand 12e anstoßen und den Deckel 12d wieder mitnehmen.

Die Seitenführung 72 ist der Deckeluntergreifungseinrichtung 71 zugeordnet und insbesondere mit dieser einstückig verbunden. Es sind insbesondere zwei Führungselemente in Form von Seitenführungsschenkeln vorgesehen, die jeweils mit einer Schiene der Deckeluntergreifungseinrichtung 71 einstückig verbunden sind. Die Seitenführung 72 ist dazu vorgesehen, dass beim Verschieben der Petrischale 12 der Petrischalen-Deckel 12d gegenüber dem Petrischalen-Unterteil 12a in der Spur bleibt, d.h. sich der Deckel 12d und das Unterteil 12a nicht in zwei unterschiedliche Horizontalrichtungen bewegen. Der lichte Abstand zwischen den beiden Seitenführungsschenkeln entspricht ungefähr dem Außendurchmesser des Petrischalen-Deckels 12d. An den Schenkeln der Seitenführung 72 kann eine Seitenführungs-Einlaufschräge vorgesehen sein, beispielsweise in Form einer Umbiegung nach außen. Beim Verschieben der Petrischale in Pfeilrichtung wird der Deckel (12d) durch Schräglage der Deckelführung 67 vom Unterteil abgehoben, so dass das Unterteil alleine weiterbeförderbar ist.

Der Niederhalter 68 erfüllt eine Doppelfunktion. Zum einen bremst er den Deckel 12d bei seiner Bewegung in der Deckel-Führung 67, zum anderen hält er den abgehobenen Deckel 12d in einer im wesentlichen senkrecht zur Bewegungsrichtung verlaufenden Richtung nieder. Der Niederhalter 68 kann ein relativ dünner Federdraht sein, der sich insbesondere mittig zwischen den beiden Seitenführungsschenkeln befindet. Gegebenenfalls ist eine Einlaufschräge in Form einer Aufbiegung des Drahts vorgesehen.

Die Bildauswerteeinrichtung 15 dient zur Bildauswertung des auf dem Nährboden der Petrischale befindlichen biologischen Materials. Die Bildauswerteeinrichtung 15 umfasst eine Kamera 73, eine lichtdurchlässige Photolichtplatte, einen Umlenkspiegel und eine Auf- und/oder Durchlichteinheit. Mit der Kamera 73 können Farbbilder und/oder Schwarz-Weiß-Bilder vom biologischen Material erstellt werden. Die Kamera 73 ist vorzugsweise mit einem Bildschirm eines Auswerterechners gekoppelt sein, so dass sofort ausgewertet werden kann. Die lichtdurchlässige Photolichtplatte befindet sich bevorzugt benachbart zur Unterteilführung 66 der Deckelabnehmeinrichtung 14, so dass das Unterteil 12a von der Unterteilführung 66 auf die Photolichtplatte gefahren werden kann. Die Auflichteinheit besitzt eine Ringleuchte und eine über der Ringleuchte angeordnete Abschirmplatte zur Lichtabschirmung nach oben.

### Verfahren

Durch die Ent- und Beladevorrichtung 13, insbesondere durch deren Transporteinrichtung 18, lässt sich die unterste Petrischale 12 aus dem Entlademagazin 16 herausnehmen und nach Gebrauch in das Belademagazin 17 zurücklegen.

Wie insbesondere in Fig. 1 dargestellt, fährt zunächst das Fahrgestell 49 für die Bewegung in x-Richtung zur Position der Positioniereinrichtung 19, in der sich das Entlademagazin 16 befindet. Das Fahrgestell 50 zur Bewegung in z-Richtung fährt auf die Positioniereinrichtung 19 zu, bis der Support 54 des Tandemschlittens 52 des Fahrgestells 51 für die Bewegung in y-Richtung unterhalb der Basis 20 des Entlagemagazins 16 im wesentlichen fluchtend zur Bodenöffnung 25 ausgerichtet ist.

Als nächstes fährt das Fahrgestell 51 für die Bewegung in y-Richtung hoch. Dabei wird der Tandemschlitten 52 nach oben bewegt und der Support 54 fährt durch die Bodenöffnung 25 in das Entlademagazin 16 ein. Die einstückig mit dem Support 54 verbundene Schiebeeinrichtung 53 hingegen bleibt außerhalb des Entlademagazins 16. Der Support 54 gelangt von der Bodenöffnung 25 nach oben in die von den Klinken 29 gebildete Durchlassöffnung 38. Beim Einfahren drückt dabei der Mittelabschnitt 59 mit seiner großen Querabmessung die vorderen, im Bereich der Seitenöffnung 27 angeordneten Klinken 29 von ihrer Schließposition 30 nach außen in ihre Öffnungsposition 31. Je weiter der Support 54 nach oben fährt, desto weiter werden die Klinken 29 nach außen zurückgedrückt. Dies wird durch die Einlaufschrägen 40 der Klinken 29 erreicht. Die Klinken 29 bewegen sich dabei mit ihrer kreisbogenförmigen Führungsnase 34 auf der Führungsbahn 35 des Basis-Unterteils 22.

Der Support 54 wird so lange nach oben gefahren, bis er eben mit den durch die Oberseiten 42 der Klinken 29 gebildeten Auflagefläche 43 abschließt. Der Boden 12d des Petrischalen-Unterteils 12a wird vom Support 54 erfasst, und die Petrischale 12 liegt nunmehr sowohl auf der Auflagefläche 43 der Klinken 29 als auch auf dem Support 54 auf. Vorher schon umgreifen die nasenförmigen Zentrierelemente 63 den Seitenrand 12c des Petrischalen-Unterteils 12a, so dass eine etwaige Fehlstellung zwischen der Petrischale 12 und dem Support 54 ausgeglichen wird.

Als nächstes wird das Fahrgestell 50 für die Bewegung in z-Richtung zurückgefahren, so dass der Support 54 mitsamt der untersten Petrischale 12 seitlich aus der Seitenöffnung 27 herausgefahren wird. Dabei werden durch den Mittelabschnitt 59 des Supports 54 die vorderen Klinken 29 noch weiter zurückgedrückt, so dass der Support 54 ausfahren kann. Die in den Öffnungsquerschnitt der Seitenöffnung 27 ragenden federförmigen Sicherungselemente 47 werden dabei durch den Petrischalen-Deckel 12d nach außen zurückgebogen, so dass sie das seitliche Entnehmen nicht behindern.

Sobald der Mittelabschnitt 59 des Supports 54 vollständig ausgefahren ist, schnappen die vorderen Klinken 29 von ihrer Öffnungsposition 31 zurück in ihre Schließposition 30. Dabei schlägt der an den Führungsnasen 34 der Klinken 29 befindliche Klinkenanschlag 37 an den Anschlag 36 an der Vorderseite der kreisbogenförmigen Führungsbahn 35 an und die Klinken 29 sind in ihrer Schließposition 30 fixiert. Der im Magazin verbleibende Stapel rutscht nach unten, so dass die jetzt unterste Petrischale auf die Oberseite der Klinken zu liegen kommt.

Der Support 54 wird so weit zurückgefahren, bis er über dem Tisch 75 ausgerichtet ist. Der Tisch 75 wird fluchtend zur mittigen Durchbrechung 61 des Supports 54 ausgerichtet. Das Fahrgestell 51 zur Bewegung in y-Richtung mitsamt dem Tandemschlitten 52 wird nach unten bewegt bis der Tisch 75 durch die Relativbewegung in die Durchbrechung 61 am Support 54 einfährt und dabei den Boden des Petrischalen-Unterteils 12a erfasst. Die Petrischale 12 ist nun auf dem Tisch 75 abgestellt. Als nächstes fährt das Fahrgestell 50 nach vorne, bis die kreisringförmige Schiebeeinrichtung 57 unterhalb des Tisches 75 liegt, wobei eine Öffnung zwischen den Ringen der Schiebeeinrichtung 53 und des Supports 54 ein Umfahren eines Tischbeins ermöglicht. Das Fahrgestell 51 und der Tandemschlitten 52 fahren nach oben. Da der lichte Durchmesser der Schiebeeinrichtung 53 größer ist als der Außendurchmesser des Petrischalen-Unterteils 12a wird das Petrischalen-Unterteil 12a nicht erfasst, sondern die Schiebeeinrichtung 53 fährt weiter, bis der Petrischalen-Deckel 12d durch Stegbereiche 55 der Schiebeeinrichtung erfasst wird. Das Petrischalen-Unterteil 12a bleibt auf dem Tisch 75 abgestellt. Der an der Schiebeeinrichtung 57 ausgebildete Vorsprung 56 in Form des Rädchens greift an den Deckelrand 12e des Petrischalendeckels 12d an und die Petrischale 12 kann seitlich verschoben werden.

Als nächstes wird das Fahrgestell 49 seitlich verschoben und gibt dabei die Schieberichtung 57 vor. Dabei wird die Petrischale 12 am Deckel 12e seitlich verschoben, so dass sie zunächst vom Tisch 75 auf die Unterteilführung 66 der Schiebeeinrichtung 53 gelangt. Das Petrischalen-Unterteil 12a liegt dabei auf der Unterteilführung 66 auf. Ein weiteres seitliches Verschieben führt dazu, dass die Deckelführung 67 erreicht wird. Die Deckeluntergreifungen 71 untergreifen die Unterkante des Deckelrandes 12e und der Deckel wird leicht angehoben und an der in Schieberichtung 57 weisenden Seite relativ vom Unterteil 12a weggekippt bzw. weggeschwenkt. Durch die Steigung der Deckelführung 67 in Schieberichtung 57 kommt es dazu, dass bei einer weiteren seitlichen Verschiebung der Öffnungswinkel zwischen dem Deckel und dem Unterteil 12a immer größer wird. Der Deckel 12d wird dabei durch die Deckeluntergreifungen 71 und seitlich durch die Seitenführungen 72 geführt und bleibt fluchtend zum Unterteil 12a ausgerichtet. Nach einem bestimmten Schiebeweg und Erreichen des Winkels zwischen Deckelführung 67 und Unterteilführung 66 wird der Deckel von Schiebevorsprung 56 abgehoben und durch den Niederhalter 68 abgebremst, so dass er nicht mehr weiter in Schieberichtung 57 seitlich verschoben werden kann. Der Deckelrand 12d ist an dieser Stelle jedoch an der in Schieberichtung 57 hinteren Seite der Petrischale 12 mit dem Seitenrand 12c des Petrischalen-Unterteils 12a noch überlappend in Kontakt.

Als nächstes wird das Fahrgestell 51 zur Bewegung in y-Richtung nach unten gefahren, so dass der Vorsprung 56, insbesondere das Rädchen, der Schiebeeinrichtung 53 jetzt am Seitenrand 12c des Petrischalen-Unterteils 12a zur Anlage kommt. Das Petrischalen-Unterteil 12a wird dann seitlich weitergeschoben, der Deckel 12d jedoch bleibt auf der Deckelführung 67 liegen. Das Petrischalen-Unterteil 12a wird mittels der Schiebeeinrichtung 53 in den Bereich der Bildauswerteeinrichtung 15 geschoben. Dabei gelangt das Petrischalen-Unterteil 12a von der Unterteilführung 66 direkt auf die lichtdurchlässige Photolichtplatte. Das Petrischalen-Unterteil 12a wird von unten durchleuchtet und von oben mittels der Ringleuchte angestrahlt. Es wird mittels der Kamera wenigstens ein Bild des auf dem Nährboden befindlichen biologischen Materials, beispielsweise von Keimen, Bakterien oder dergleichen aufgenommen. Als nächstes erfolgt eine Auswertung anhand der Bilder.

Nach der Bildauswertung wird das Petrischalen-Unterteil 12a durch die Schiebeeinrichtung 53 entgegen der ursprünglichen Schieberichtung 57 zurück in den Bereich der Deckelabnehmeinrichtung 14 geschoben. Der Seitenrand 12c des Petrischalen-Unterteils 12a erfasst den Deckelrand 12e des Petrischalen-Deckels 12d, so das der Deckel 12d mitgenommen wird und wieder lose auf dem Petrischalen-Unterteil 12a zu liegen kommt.
Als nächstes erfolgt ein Umsetzen der Petrischale 12 von der Schiebeeinrichtung 57 auf den Tisch 75 und wird dann wieder von dem Support 54 übernommen analog der zuvor beschriebenen Weise.

Das Fahrgestell 49 fährt auf die Höhe des Belademagazins 17. Als nächstes fährt das Fahrgestell 50 nach vorne, so dass der Support mitsamt der Petrischale 12 unterhalb der Bodenöffnung 25 des Belademagazins 17 zu liegen kommt. Das Fahrgestell 51 mitsamt dem Tandemschlitten 52 fährt nach oben, so dass die Petrischale 12 und der Support 54 durch die Bodenöffnung 25 in das Belademagazin 16 gelangen. Von der Bodenöffnung 25 gelangt die Petrischale 12 in die durch die Klinken 29 gebildete Durchlassöffnung 38. Dabei werden alle vier Klinken 29 durch den Deckel 12d der Petrischale 12 von ihrer Schließposition 30 in ihre Öffnungsposition 31 gedrückt. Befinden sich im Belademagazin 17 Petrischalen, werden diese durch die eingeführte Petrischale 12 nach oben gedrückt. Der Support 54 mitsamt der Petrischale 12 fährt so lange nach oben, bis er eben mit der durch die Klinken 29 gebildeten Auflagefläche 43 abschließt. Dabei schnappen die Klinken 29 ein bestimmtes Stück in Richtung Schließposition 30 bis zum Anschlag an den Seitenflächen des Support zurück, so dass die eingeführte Petrischale 12 untergriffen wird. Das Fahrgestell 51 fährt nach unten, so dass der Support 54 aus dem Belademagazin 17 herausfährt. Dabei schnappen die Klinken 29 vollständig in ihre Schließposition 30 zurück. Die Petrischale 12 ist im Belademagazin 17 abgelegt.

## Patentansprüche

1. Verfahren zum Ent- und Beladen wenigstens eines mit mindestens einer Petrischale bestückbaren Magazins, wobei die Petrischale ein einen Boden und einen zylindrischen Seitenrand aufweisendes schalenförmiges Unterteil und einen lose darauf liegenden Deckel besitzt, das Verfahren mit folgenden Schritten:
- Seitliches Entnehmen einer untersten Petrischale aus einem ersten Magazin wobei das Unterteil der Petrischale mindestens an ihrem Boden erfasst wird;
- Einführen der Petrischale nach Gebrauch von unten durch einen offenen Magazinboden des ersten oder eines anderen Magazins

2. Verfahren nach Anspruch 1, **dadurch gekennzeichnet, dass** die Petrischale zum Entnehmen an der Seitenwand erfasst wird.

3. Verfahren nach Anspruch 1 oder 2, **dadurch gekennzeichnet, dass** ein Support eines Schlittens zum Entnehmen der Petrischale von unten durch den offenen Magazinboden einfährt und dabei vorzugsweise Einlaufschrägen aufweisende, öffenbare Sperren öffnet und diese nach der Entnahme wieder zurückschnappen.

4. Verfahren nach einem der vorhergehenden Ansprüche, **dadurch gekennzeichnet, dass** die Petrischale, insbesondere ihr Deckel beim Einführen ins Magazin von unten die Sperren öffnet und diese nach dem Einführen wieder zurückschnappen.

5. Verfahren nach einem der vorhergehenden Ansprüche, **dadurch gekennzeichnet, dass** die Sperren im wesentlichen in radialer Richtung des Magazins nach außen gedrückt werden und nach innen zurückschnappen.

6. Verfahren nach einem der vorhergehenden Ansprüche, **dadurch gekennzeichnet, dass** die Petrischale nach dem Entnehmen auf einen durch eine Öffnung des Supports passenden, den Boden der Petrischale erfassenden Tisch abgesetzt wird.

7. Verfahren nach einem der vorhergehenden Ansprüche, **dadurch gekennzeichnet, dass** die Petrischale von einer den Deckel erfassenden Schiebeeinrichtung seitlich verfahren, insbesondere verschoben wird.

8. Verfahren nach einem der vorhergehenden Ansprüche, **dadurch gekennzeichnet, dass** die Petrischale nach dem Entnehmen, vorzugsweise nach einem Abheben des Deckels in eine Position zum Gebrauch, insbesondere zur Bildauswertung gefahren wird.

9. Verfahren nach einem der vorhergehenden Ansprüche, **dadurch gekennzeichnet, dass** das erste Magazin mit mehreren Petrischalen beladen ist und eine Petrischale nach Gebrauch von unten in das zweite Magazin eingeführt wird.

10. Verfahren nach einem der vorhergehenden Ansprüche, **dadurch gekennzeichnet, dass** zum Einführen der Schlitten unters zweite Magazin fährt.

11. Be- und Entladevorrichtung für Petrischalen, insbesondere zur Durchführung des Verfahrens nach einem der Ansprüche 1 bis 10, wobei die Petrischale (12) ein einen Boden (12b) und einen zylindrischen Seitenrand (12c) aufweisendes schalenförmiges Unterteil (12a) und einen lose darauf liegenden Deckel (12d) besitzt mit wenigstens einem Magazin (16, 17), das mit Petrischalen (12) bestückbar ist und einem Schlitten (52) mit wenigstens einem Support (54) zur Aufnahme der Petrischale (12), wobei das Magazin (16, 17) eine Bodenöffnung (25) aufweist mit einem lichten Durchmesser, der größer ist als der Außendurchmesser des Supports (54) und der Petrischale (12) und zumindest in Höhe der untersten Petrischale (12) eine Seitenöffnung (27) aufweist deren Höhenabmessung in axialer Richtung des Magazins (16, 17) größer ist als die Gesamthöhe aus Petrischale (12) und Support (54).

12. Vorrichtung nach Anspruch 11, **dadurch gekennzeichnet, dass** das Magazin (16, 17) Sperren aufweist die schenkbeweglich zwischen einer Öffnungs- und Schließposition (30, 31) am Magazin (16, 17) gelagert sind und eine axial in einer Einführrichtung gesehen verjüngende Durchlassöffnung (38) für die Petrischalen (12) bilden, wobei der lichte Durchmesser der Durchlassöffnung (38) in Schließposition (30) der Sperren größer ist als der Außendurchmesser des Supports (54), wobei die Sperren vorzugsweise als Klinken (29) ausgebildet sind.

13. Vorrichtung nach Anspruch 12, **dadurch gekennzeichnet, dass** die Klinken (29) eine Auflagefläche (43) für die unterste Petrischale (12) des Magazins (16, 17) bilden, die vorzugsweise durch ebene, im wesentlichen senkrecht zu einer Magazinlängsachse (39) ausgerichtete Oberseiten (42) der Klinken (29) gebildet wird.

14. Vorrichtung nach einem der Ansprüche 12 oder 13, **dadurch gekennzeichnet, dass** die Sperren Rückstellelemente (32), insbesondere Federelemente aufweisen, die ein selbsttätiges Zurückschnappen in die Schließposition (30) ermöglichen.

15. Vorrichtung nach einem der Ansprüche 11 bis 14, **dadurch gekennzeichnet, dass** das Magazin (16, 17) wenigstens ein Sicherungselement (47) zur Sicherung der Petrischale (12) gegen seitliches Herausfallen aus der Seitenöffnung (27) aufweist.

16. Vorrichtung nach einem der Ansprüche 11 bis 15, **dadurch gekennzeichnet, dass** der Innendurchmesser des Deckels (12d) größer ist, als der Außendurchmesser des Petrischalen-Unterteils (12a), vorzugsweise das Verhältnis des Deckelinnendurchmessers zum Unterteilaußendurchmesser im Bereich von 1,01 bis 1,06, insbesondere 1,03 bis 1,05 liegt.

17. Vorrichtung nach einem der Ansprüche 11 bis 16, **dadurch gekennzeichnet, dass** der Support (54) des Schlittens (52) Außenabmessungen aufweist, die kleiner sind als der lichte Öffnungsquerschnitt der Bodenöffnung (25) des Magazins (16, 17) und größer sind als die von den Sperren in Schließposition (30) gebildete Durchlassöffnung (38) an einer bestimmten Höhe der Sperren und wobei sich beim Support (54) an einen Abschnitt großer Breite ein Abschnitt mit gegenüber der großen Breite kleineren Querabmessung anschließt, wobei diese kleine Querabmessung kleiner ist, als die von den Sperren gebildete seitliche Durchlassöffnung (38) und wobei vorzugsweise der Support (54) eine mittige Durchbrechung (61) aufweist, insbesondere der Support (54) als relativ schmales Materialband ausgebildet ist, das eine äußere und eine die Durchbrechung definierende innere Kontur bildet.

18. Vorrichtung nach einem der Ansprüche 11 bis 17, **dadurch gekennzeichnet, dass** der Support (54) Zentrierelemente (63) zur Sicherung der Petrischale 12 gegen seitliches Abrutschen vom Support (54) aufweist.

19. Vorrichtung nach einem der Ansprüche 11 bis 18, **dadurch gekennzeichnet, dass** eine Schiebeeinrichtung (53) vorgesehen ist, mit in einer Schieberichtung (57) gegenüberliegenden Randbegrenzungen, insbesondere Zentrierungen, deren lichter Abstand größer ist als der Außendurchmesser der Petrischale (12) und kleiner ist als der Außendurchmesser des Deckels (12d).

20. Vorrichtung nach einem der Ansprüche 11 bis 19, **dadurch gekennzeichnet, dass** im Bereich der Randbegrenzungen ein Vorsprung (56) vorgesehen ist, der in den Abstandsbereich der Randbegrenzungen hineinragt.

21. Vorrichtung nach einem der Ansprüche 11 bis 20, **dadurch gekennzeichnet, dass** ein Tisch zum Absetzen der Petrischale (12) vorgesehen ist, dessen größte Außenabmessung kleiner ist als die größte Außenabmessung der Durchbrechung des Supports (54) und kleiner ist als der lichte Abstand der Randbegrenzungen der Schiebeeinrichtung (53).

22. Vorrichtung nach einem der Ansprüche 11 bis 21, **dadurch gekennzeichnet, dass** eine Deckelabnehmeinrichtung (14) vorgesehen ist.

23. Vorrichtung nach einem der Ansprüche 11 bis 22, **dadurch gekennzeichnet, dass** eine Gebrauchseinrichtung zum Gebrauch der Petrischale (12) vorgesehen ist, die vorzugsweise eine Bildauswerteeinrichtung (15) ist, insbesondere mit einer lichtdurchlässigen Fotolichtplatte, einer Kamera (73) und einer Auf- und/oder Durchlichteinheit aufweist.

24. Vorrichtung nach einem der Ansprüche 11 bis 23, **dadurch gekennzeichnet, dass** das Magazin (16, 17) mittels einer Positioniereinrichtung (19) lösbar an der Vorrichtung angeordnet ist.
